(19) Europäisches Patentamt / European Patent Office / Office européen des brevets

(11) EP 4 555 921 A1

(12) EUROPEAN PATENT APPLICATION
published in accordance with Art. 153(4) EPC

(43) Date of publication:
21.05.2025 Bulletin 2025/21

(51) International Patent Classification (IPC):
*A61B 5/00* (2006.01)

(21) Application number: 23839931.5

(52) Cooperative Patent Classification (CPC):
A61B 5/00

(22) Date of filing: 11.07.2023

(86) International application number:
PCT/KR2023/009860

(87) International publication number:
WO 2024/014839 (18.01.2024 Gazette 2024/03)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 11.07.2022 KR 20220084815

(71) Applicant: Lululab Inc.
Seoul 06054 (KR)

(72) Inventors:
• CHOE, Yongjoon
  Seoul 06305 (KR)
• JUNG, Geunho
  Seoul 06041 (KR)
• LEE, Jong Ha
  Hwaseong-si, Gyeonggi-do 18456 (KR)
• YOO, Sangwook
  Seoul 07986 (KR)

(74) Representative: Franke, Dirk
Franke & Partner
Patent- und Rechtsanwälte
Widenmayerstraße 25
80538 München (DE)

(54) **METHOD AND DEVICE FOR ANALYZING SKIN COMPONENT AMOUNT BY USING MULTIPLE WAVELENGTHS**

(57) According to various embodiments, a component amount measurement device for measuring skin component amount information about a user comprises: a skin measurement unit for measuring the skin of a user; and a skin component amount calculation unit for calculating the component amount for a target component in the skin of the user, wherein the skin measurement unit comprises a light-emitting unit that includes at least one light source and a light detection unit that includes at least one light detector, the light-emitting unit includes a first light source for irradiating the surface of the skin with a first light having a plurality of wavelengths, the light detection unit includes a first light detector for detecting the first light irradiated from the first light source, and the skin component amount calculation unit measures the component amount for the target component on the basis of a light signal of the first light absorbed into the skin and scattered thereon.

Fig. 2

## Description

### Technical Field

The present disclosure relates to a method and a device for analyzing a skin component amount by using multiple wavelengths and, more particularly, to a method and a device for measuring a skin component amount inside the skin by using a multi-wavelength light source and a light detector.

### Background Art

Unless otherwise indicated herein, the matters described in this section are not prior art to the claims of this application and their inclusion in this section is not an admission that they are prior art.

In recent years, interest in skin health, especially facial skin health, has been increasing. As interest in skin health increases, various cosmetics and beauty devices are appearing. In particular, a skin condition measurement method is being developed that captures images of the facial skin of a user and analyzes various skin troubles (e.g., wrinkles, pores, acne, etc.) on the face of the user. Such a method involves obtaining data on skin components by using a specific light source.

In the diffuse reflectance spectroscopy (DRS) method, which is generally used for research purposes abroad, light is mainly emitted from a tungsten-halogen lamp light source and a spectrometer as a light detector via an optical fiber is used to simultaneously measure a wide wavelength range.

However, the aforementioned method cannot obtain spatial information as it is a single-point measurement method, is inefficient as it measures an entire wavelength band, including wavelengths not used for fitting, and makes miniaturization difficult. Therefore, a method that can be utilized in various fields needs to be devised.

### Disclosure

### Technical Problem

In order to solve the above problems, the objective of the present disclosure is to provide a method and a device for measuring a skin component amount inside the skin by using a multi-wavelength light source (e.g., a white light LED light source) and a light detector.

### Technical Solution

According to various embodiments, a component amount measurement device for measuring skin component amount information about a user includes: a skin measurement unit for measuring the skin of the user; and a skin component amount calculation unit for calculating a component amount for a target component in the skin of the user, wherein the skin measurement unit includes: a light-emitting unit that includes at least one light source; and a light detection unit that includes at least one light detector, wherein the light-emitting unit includes a first light source for irradiating a surface of the skin with a first light having a plurality of wavelengths; and the light detection unit includes a first light detector for detecting the first light emitted from the first light source, wherein the skin component amount calculation unit measures the component amount for the target component on the basis of a light signal of the first light absorbed and scattered within the skin.

According to various embodiments, light emitted from the at least one light source may be LED white light, the first light detector may include a plurality of bandpass filters arranged in a grid arrangement, and may obtain a light signal for each of the plurality of wavelengths included in the first light through the plurality of bandpass filters, and the skin component amount calculation unit calculates the component amount for the target component on the basis of the light signal for each of the plurality of wavelengths.

According to various embodiments, the skin component amount calculation unit may calculate the component amount for the target component on the basis of at least concentration of each component in the skin, extinction coefficient of each component in the skin, and a distance between the first light source and the first light detector.

According to various embodiments, the at least one light source may include the first light source and a second light source arranged to be adjacent to the first light source, and the at least one light detector may include the first light detector and a second light detector arranged to be adjacent to the first light detector, the skin component amount calculation unit may calculate a first component amount for the target component within a first skin region between the first light source and the first light detector through the first light source and the first light detector, calculate a second component amount for the target component within a second skin region between the first light source and the second light detector through the first light source and the second light detector, calculate a third component amount for the target component within a third skin region between the second light source and the first light detector through the second light source and the first light detector, and calculate a fourth component amount for the target component within a fourth skin region between the second light source and the second light detector through the second light source and the second light detector.

According to various embodiments, the skin component amount calculation unit may determine a fifth skin region excluding the first skin region to the fourth skin region, calculate a fifth component amount for the target component within the fifth skin region on the basis of the first component amount to the fourth component amount, and generate a two-dimensional image map for the target component to generate spatial information for the target component.

**Advantageous Effects**

According to various embodiments disclosed in this document, the aim is to measure absolute quantities, thereby providing high usability for diagnostic and therapeutic purposes compared to existing relative value measurement methods.

In addition, according to various embodiments, a relatively low-cost and miniaturized system is used compared to an existing system that uses a modulated light source aimed at measuring absolute values, thereby enabling application to wearable devices in the future.

In addition, according to various embodiments, a two-dimensional distribution map of skin components is provided, thereby enabling utilization as medical data for effective skin beauty, clinical diagnosis, and treatment.

In addition, according to various embodiments, there is applicability in various fields, such as using optical characteristic data of components of fruits, foods, etc. instead of the skin.

In addition, various effects that are directly or indirectly identified through this document may be provided.

**Description of Drawings**

FIG. 1 is a block diagram of a component amount measurement device within a network environment according to various embodiments.

FIG. 2 is a diagram illustrating main components of the device according to an embodiment.

FIG. 3 is a drawing illustrating a light-emitting unit and a light detection unit in contact with the skin according to an embodiment.

FIG. 4 is a diagram illustrating the arrangement of the light-emitting unit and the light detection unit of a skin measurement unit according to an embodiment.

FIG. 5 is a diagram illustrating a region in which skin component data is detected according to the arrangement of the light-emitting unit and the light detection unit.

FIG. 6 is a diagram illustrating the configuration of the hardware of the device according to FIG. 1.

**Mode for Invention**

The present disclosure may have various modifications and embodiments, and specific embodiments are illustrated in the drawings and described in detail in the detailed description. However, this is not intended to limit the present disclosure to a specific embodiment, but should be understood to include all modifications, equivalents or substitutes included in the spirit and technical scope of the present disclosure. In describing each drawing, similar reference numerals are used to refer to similar components.

Terms such as "first", "second", "A", "B", etc. may be used to describe various components, but such components should not be limited by such terms. The terms are used solely to distinguish one component from another. For example, without exceeding the scope of the present disclosure, the first component may be named the second component, and similarly, the second component may be named the first component. The term "and/or" includes any combination of a plurality of related described items or any one of a plurality of related described items.

When it is mentioned that a component is "connected" or "coupled" to another component, it should be understood that the component may be directly connected or coupled to the another component, but there may be other components present therebetween. On the other hand, when it is mentioned that a component is "directly connected" or "directly coupled" to another component, it should be understood that there are no other components therebetween.

The terminology used in this application is for the purpose of describing particular embodiments only and is not intended to limit the present disclosure. Singular expressions include plural expressions unless the context clearly indicates otherwise. In this application, it should be understood that terms such as "include" or "have" are intended to specify the presence of a feature, number, step, operation, component, part or combination thereof described in the specification, but do not exclude in advance the possibility of the presence or addition of one or more other features, numbers, steps, operations, components, parts or combinations thereof.

Unless otherwise defined, all terms used herein, including technical or scientific terms, have the same meanings as commonly understood by those skilled in the art to which the present disclosure belongs. Terms defined in commonly used dictionaries should be interpreted as having meanings consistent with their meanings in the context of the relevant art, and should not be interpreted in idealized or overly formal sense unless expressly defined in this application.

Hereinafter, preferred embodiments according to the present disclosure will be described in detail with reference to the attached drawings.

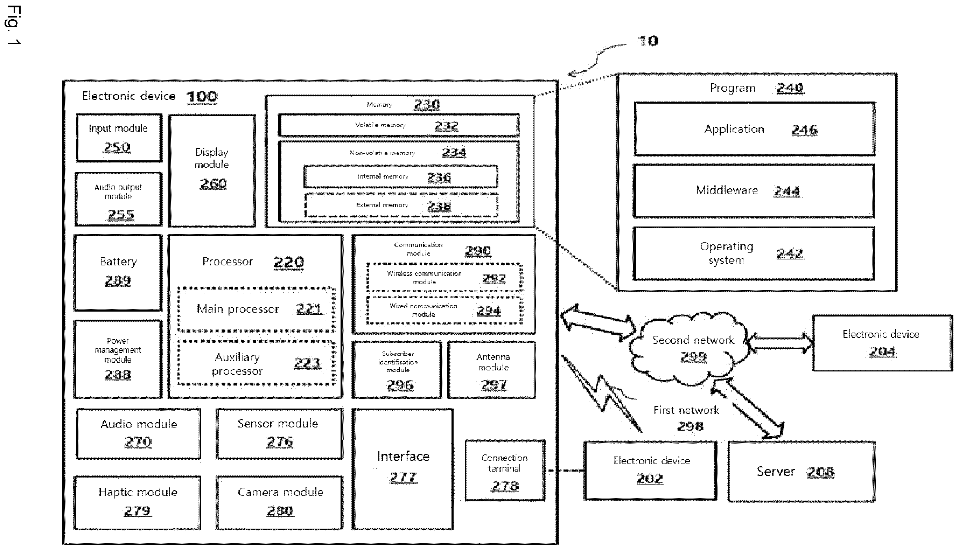

FIG. 1 is a block diagram of a component amount measurement device 100 within a network environment 10 according to various embodiments. Referring to FIG. 1, in the network environment 10, the component amount measurement device 100 may communicate with an electronic device 202 through a first network 298 (e.g., a short-range wireless communication network), or with an electronic device 204 or a server 208 through a second network 299 (e.g., a long-range wireless communication network). According to an embodiment, the component amount measurement device 100 may communicate with the electronic device 204 through the server 208. According to an embodiment, the component amount measurement device 100 may include a processor 220, a memory 230, an input module 250, an audio output module 255, a display module 260, an audio module 270, a sensor module 276, an interface 277, a connection terminal 278, a haptic module 279, a camera module 280, a power management module 288, a battery 289, a communication module 290, a subscriber identification module 296, or an antenna module 297. In an embodiment, the component amount measurement device 100 may have at least one of these components (e.g., the connection terminal 278) omitted, or one or more other components added. In an embodiment, some of these components (e.g., the sensor module 276, the camera module 280, or the antenna module 297) may be integrated into a single component (e.g., the display module 260) .

The processor 220 may, for example, execute software (e.g., a program 240) to control at least one other component (e.g., a hardware or software component) of the component amount measurement device 100 connected to the processor 220 and perform various data processing or calculations. According to an embodiment, as at least part of data processing or calculation, the processor 220 may store instructions or data received from another component (e.g., the sensor module 276 or the communication module 290) in a volatile memory 232, process the instructions or data stored in the volatile memory 232, and store resulting data in a non-volatile memory 234. According to an embodiment, the processor 220 may include a main processor 221 (e.g., a central processing unit or an application processor) or an auxiliary processor 223 (e.g., a graphics processing unit, a neural processing unit (NPU), an image signal processor, a sensor hub processor, or a communication processor) that may operate independently or together therewith. For example, when the component amount measurement device 100 includes the main processor 221 and the auxiliary processor 223, the auxiliary processor 223 may be configured to use lower power than the main processor 221 or to be specialized for a designated function. The auxiliary processor 223 may be implemented separately from the main processor 221, or as a part thereof.

For example, the auxiliary processor 223 may control at least a portion of functions or states associated with at least one of the components of the component amount measurement device 100 (e.g., the display module 260, the sensor module 276, or the communication module 290) on behalf of the main processor 221 while the main processor 221 is in an inactive state (e.g., sleep), or together with the main processor 221 while the main processor 221 is in an active state (e.g., running an application). According to an embodiment, the auxiliary processor 223 (e.g., the image signal processor or the communication processor) may be implemented as part of another functionally related component (e.g., the camera module 280 or the communication module 290). According to one embodiment, the auxiliary processor 223 (e.g., a neural network processing unit) may include a hardware structure specialized for processing an artificial intelligence model. The artificial intelligence model may be generated through machine learning. This learning may be performed, for example, in the component amount measurement device 100 in which artificial intelligence is performed, or may be performed through a separate server (e.g., the server 208). A learning algorithm may, for example, include supervised learning, unsupervised learning, semi-supervised learning, or reinforcement learning, but is not limited to the examples described above. The artificial intelligence model may include a plurality of artificial neural network layers. An artificial neural network may be one of a deep neural network (DNN), a convolutional neural network (CNN), a recurrent neural network (RNN), a restricted Boltzmann machine (RBM), a deep belief network (DBN), a bidirectional recurrent deep neural network (BRDNN), a deep Q-network, and a combination of two or more of the above, but is not limited to the examples described above. The artificial intelligence model may include a software structure in addition to or as an alternative to the hardware structure.

The memory 230 may store various data used by at least one component of the component amount measurement device 100 (e.g., the processor 220 or the sensor module 276). The data may include, for example, input data or output data for software (e.g., the program 240) and instructions associated therewith. The memory 230 may include the volatile memory 232 or the non-volatile memory 234.

The program 240 may be stored as software in the memory 230 and may include, for example, an operating system 242, middleware 244, or an application 246.

The input module 250 may receive instructions or data to be used by a component (e.g., the processor 220) of the component amount measurement device 100 from the outside (e.g., a user) of the component amount measurement device 100. The input module 250 may include, for example, a microphone, a mouse, a keyboard, keys (e.g., buttons), or a digital pen (e.g., a stylus pen).

The audio output module 255 may output an audio signal to the outside of the component amount measurement device 100. The audio output module 255 may include, for example, a speaker or a receiver. The speaker may be used for general purposes such as playing multimedia or playing recordings. The receiver may be used to receive incoming calls. According to an embodiment, the receiver may be implemented separately from the speaker, or as part of the speaker.

The display module 260 may visually provide information to an external party (e.g., a user) of the component amount measurement device 100. The display module 260 may include, for example, a display, a holographic device, or a projector and a control circuit for controlling the corresponding device. According to an embodiment, the display module 260 may include a touch sensor configured to detect a touch, or a pressure sensor configured to measure a magnitude of a force generated by the touch.

The audio module 270 may convert sound into an electrical signal, or may convert an electrical signal into sound. According to an embodiment, the audio module 270 may acquire sound through the input module 250, or output sound through the audio output module 255, or an external electronic device (e.g., the electronic device 202) (e.g., a speaker or headphones) directly or wirelessly connected to the component amount measurement device 100.

The sensor module 276 may detect an operating status (e.g., power or temperature) of the component amount measurement device 100 or an external environmental status (e.g., status of a user) and generate an electrical signal or data value corresponding to the detected status. According to an embodiment, the sensor module 276 may include, for example, a gesture sensor, a gyro sensor, a barometric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 277 may support one or more designated protocols that can be used to connect the component amount measurement device 100 directly or wirelessly with the external electronic device (e.g., the electronic device 202). According to an embodiment, the interface 277 may include, for example, a high definition multimedia interface (HDMI), a universal serial bus (USB) interface, an SD card interface, or an audio interface.

The connection terminal 278 may include a connector through which the component amount measurement device 100 may be physically connected to the external electronic device (e.g., the electronic device 202). According to an embodiment, the connection terminal 278 may include, for example, an HDMI connector, a USB connector, an SD card connector, or an audio connector (e.g., a headphone connector).

The haptic module 279 may convert electrical signals into mechanical stimuli (e.g., vibration or movement) or electrical stimuli that can be perceived by a user through tactile or kinesthetic sensations. In an embodiment, the haptic module 279 may include, for example, a motor, a piezoelectric element, or an electrical stimulation device.

The camera module 280 may capture still images and videos. According to an embodiment, the camera module 280 may include one or more lenses, image sensors, image signal processors, or flashes.

The power management module 288 may manage power supplied to the component amount measurement device 100. According to an embodiment, the power management module 288 may be implemented, for example, as at least part of a power management integrated circuit (PMIC).

The battery 289 is capable of powering at least one component of the component amount measurement device 100. In an embodiment, the battery 289 may include, for example, a non-rechargeable primary battery, a rechargeable secondary battery, or a fuel cell.

The communication module 290 may support establishing a direct (e.g., wired) communication channel or a wireless communication channel between the component amount measurement device 100 and the external electronic device (e.g., the electronic device 202, the electronic device 204, or the server 208), and performing communication through the established communication channel. The communication module 290 may operate independently of the processor 220 (e.g., an application processor) and may include one or more communication processors supporting direct (e.g., wired) or wireless communication. According to an embodiment, the communication module 290 may include a wireless communication module (292) (e.g., a cellular communication module, a short-range wireless communication module, or a global navigation satellite system (GNSS) communication module) or a wired communication module 294 (e.g., a local area network (LAN) communication module, or a power line communication module). A corresponding communication module of these communication modules may communicate with the external electronic device 204 through the first network 298 (e.g., a short-range communication network such as Bluetooth, WiFi (wireless fidelity) direct, or IrDA (infrared data association)) or the second network 299 (e.g., a long-range communication network such as a legacy cellular network, a 5G network, a next-generation communication network, the Internet, or a computer network (e.g., a LAN or WAN)). These different types of communication modules may be integrated into a single component (e.g., a single chip) or implemented as a plurality of separate components (e.g., a plurality of chips). The wireless communication module 292 may identify or authenticate the component amount measurement device 100 within a communication network, such as the first network 298 or the second network 299, by using subscriber information (e.g., international mobile subscriber identity (IMSI)) stored in the subscriber identification module 296.

The wireless communication module 292 may support a 5G network and next-generation communication technology after a 4G network, such as NR access technology (new radio access technology). NR access technology may support high-speed transmission of high-capacity data (eMBB (enhanced mobile broadband)), minimizing terminal power and connecting multiple terminals (mMTC (massive machine type communications)), or high reliability and low latency (URLLC (ultra-reliable and low-latency communications)). The wireless communication module 292 may support high frequency bands (e.g., mmWave bands) to achieve high data transfer rates, for example. The wireless communication module 292 may support various technologies for securing performance in a high-frequency band, such as beamforming, massive multiple-input and multiple-output (MIMO), full dimensional MIMO (FD-MIMO), array antenna, analog beam-forming, or large scale antenna. The wireless communication module 292 may support various requirements specified for the component amount measurement device 100, the external electronic device (e.g., the electronic device 204), or the network system (e.g., the second network 299). According to an embodiment, the wireless communication module 292 may support a peak data rate (e.g., 20 Gbps or higher) for eMBB realization, a loss coverage (e.g., 164 dB or lower) for mMTC realization, or a U-plane latency (e.g., 0.5 ms or lower for each of downlink (DL) and uplink (UL), or 1 ms or lower for round trip) for URLLC realization.

The antenna module 297 may transmit or receive signals or power to or from an external device (e.g., the external electronic device). According to an embodiment, the antenna module 297 may include an antenna including a radiator formed of a conductor or conductive pattern formed on a substrate (e.g., a PCB). According to an embodiment, the antenna module 297 may include a plurality of antennas (e.g., an array antenna). In this case, at least one antenna suitable for a communication method used in a communication network, such as the first network 298 or the second network 299, may be selected from the plurality of antennas, for example, by the communication module 290. A signal or power may be transmitted or received between the communication module 290 and the external electronic device through the selected at least one antenna. According to an embodiment, another component (e.g. a radio frequency integrated circuit (RFIC)) in addition to the radiator may be additionally formed as part of the antenna module 297.

According to various embodiments, the antenna module 297 may form an mmWave antenna module. In an embodiment, an mmWave antenna module may include a printed circuit board, an RFIC positioned on or adjacent to a first surface (e.g., a lower surface) of the printed circuit board and capable of supporting a designated high frequency band (e.g., a mmWave band), and a plurality of antennas (e.g., an array antenna) positioned on or adjacent to a second surface (e.g., a upper surface or a side surface) of the printed circuit board and capable of transmitting or receiving signals in the designated high frequency band.

At least some of the above components may be interconnected and exchange signals (e.g., instructions or data) with each other through a communication method between peripheral devices (e.g., a bus, a general purpose input and output (GPIO), a serial peripheral interface (SPI), or a mobile industry processor interface (MIPI)).

According to an embodiment, instructions or data may be transmitted or received between the component amount measurement device 100 and the external electronic device 204 through the server 208 connected to the second network 299. Each of the external electronic devices 202 or 204 may be the same type of device as or a different type of device from the component amount measurement device 100. According to an embodiment, all or part of operations performed in the component amount measurement device 100 may be performed in one or more external electronic devices of the external electronic devices 202, 204, or 208. For example, when the component amount measurement device 100 is required to perform a function or service automatically or in response to a request from a user or another device, the component amount measurement device 100 may, instead of or in addition to executing the function or service itself, request one or more of external electronic devices to perform at least a portion of the function or service. The one or more external electronic devices receiving the request may execute at least a portion of the requested function or service, or an additional function or service related to the request, and transmit the result of the execution to the component amount measurement device 100. The component amount measurement device 100 may process the result either as it is or additionally, and may provide the result as at least a portion of the response to the request. For this purpose, for example, cloud computing, distributed computing, mobile edge computing (MEC), or client-server computing technology may be used. The component amount measurement device 100 may provide ultra-low latency services, for example, by using distributed computing or mobile edge computing. In another embodiment, the external electronic device 204 may include an Internet of Things (IoT) device. The server 208 may be an intelligent server utilizing machine learning and/or neural networks. According to an embodiment, the external electronic device 204 or the server 208 may be included within the second network 299. The component amount measurement device 100 may be applied to an intelligent service (e.g., a smart home, a smart city, a smart car, or healthcare) based on 5G communication technology and IoT-related technology.

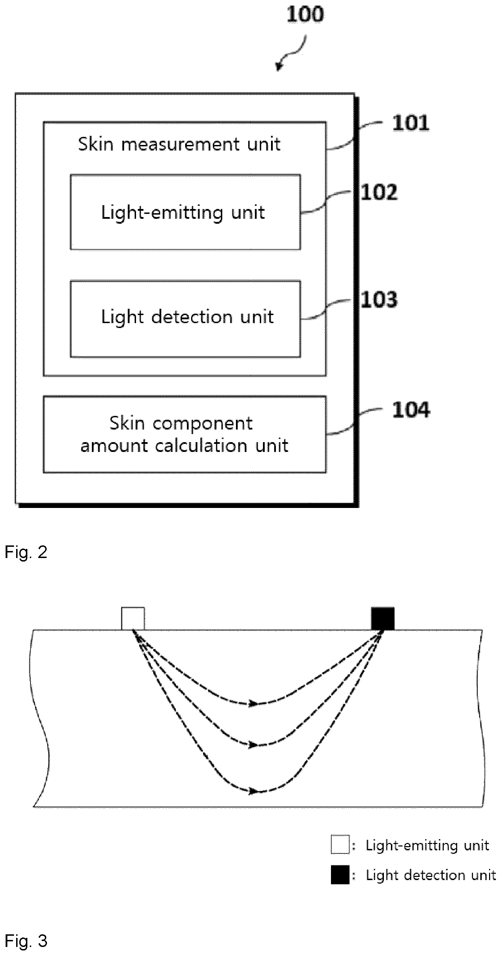

FIG. 2 is a diagram illustrating main components of the component amount measurement device 100.

The component amount measurement device 100 may include a skin measurement unit 101 and a skin component amount calculation unit 104. The skin measurement unit 101 may include a light-emitting unit 102 and a light detection unit 103. The light-emitting unit 102 may include a plurality of light sources, and the light detection unit 103 may include a plurality of light detectors. Light emitted from the light-emitting unit 102 may be incident on the skin, reflected therefrom, and detected by the light detection unit 103. In other words, some of the emitted light may be absorbed or scattered by various components of the skin (e.g., water, hemoglobin, melanin, fat, etc.), and the remaining part of the light after being absorbed or scattered may be detected by the light detection unit 103.

The skin measurement unit 101 may obtain component data for a specific component through at least one light source and at least one light detector. The skin component amount calculation unit 104 may calculate a component amount on the basis of the component data for the specific component (e.g., melanin) obtained through the skin measurement unit 101. The skin component amount calculation unit 104 may calculate the component amount for the specific component on the basis of the component data measured in real time from the skin measurement unit 101.

The skin measurement unit 101 may measure a light signal (e.g., light quantity and spectral distribution) emitted from a light source by using a reflectance plate or an integrating sphere. The skin measurement unit 101 may measure the amount of light received through the light detection unit 103. The light detection unit 103 may measure the light signal of the light emitted from the light source minus energy lost to absorption and/or scattering within the skin. The light detection unit 103 may output an electrical signal obtained by photoelectric conversion of a light signal input to the light detection unit 103. In other words, each of the light detectors may detect a light signal input through the skin as an electrical signal. The light detection unit 103 may be understood as a sensor capable of detecting a plurality of wavelengths simultaneously. For example, the light detection unit 103 may include a plurality of bandpass filters arranged in a grid arrangement.

The skin component amount calculation unit 104 may measure the component amount of a specific component in the skin on the basis of light signal information acquired through the light-emitting unit 102 and the light detection unit 103. In other words, the skin component amount calculation unit 104 may measure the component amount of a specific component in the skin on the basis of light signal information acquired through the plurality of light sources and the plurality of light detectors. Each of the plurality of light sources may emit LED white light, and the LED white light may include multiple wavelengths. For example, the LED white light may include a combination of red LED light, blue LED light, and/or green LED light. Alternatively, the LED white light may include a combination of LED lights having multiple wavelengths in addition to red LED light, blue LED light, and green LED light. Alternatively, the LED white light may include a combination of blue LED light and a yellow phosphor.

The skin component amount calculation unit 104 may calculate the amount of a target component on the basis of concentration of each target component, absorption coefficient (or extinction coefficient) of each target component, and a distance between the light source and the light detector.

Specifically, the skin component amount calculation unit 104 may calculate the degree of loss due to absorption and scattering inside the skin by wavelength by comparing a light source irradiating the skin with a light detection spectrum scattered from the skin. The skin component amount calculation unit 104 may set the amount of target components to be calculated among the skin components as an unknown number, and may calculate the skin component amount on the basis of an equation regarding the degree of energy loss due to absorption and/or scattering corresponding to one wavelength inside the skin.

In an embodiment, the skin component amount calculation unit 104 may calculate the degree of light scattering. The skin component amount calculation unit 104 may calculate the degree of light scattering on the basis of Rayleigh scattering and/or Mie scattering. The skin component amount calculation unit 104 may calculate a degree of total light scattering through the following Mathematical expression 1.

[Mathematical expression 1]

$$\mu_s'(\lambda) = a\left(f_{Ray}\left(\frac{\lambda}{\lambda_0}\right)^{-4} + (1 - f_{Ray})\left(\frac{\lambda}{\lambda_0}\right)^{-b_{Mie}}\right)$$

In Mathematical expression 1 above, $f_{Ray}$ may represent a degree to which Rayleigh scattering occurs, a may represent scattering amplitude (reduced scattering coefficient at wavelength $\lambda_0$), $b_{Mie}$ may represent scattering power (a constant related to a particle size), and $\lambda_0$ may represent a reference wavelength.

In an embodiment, the skin component amount calculation unit 104 may calculate the degree of light absorption. The skin component amount calculation unit 104 may calculate the degree of light absorption on the basis of the absorption coefficient of each component and the concentration of each component. The skin component amount calculation unit 104 may calculate the degree of light absorption through Mathematical expression 2 below according to Beer's law.

[Mathematical expression 2]

$$\mu_a(\lambda) = \Sigma_i \varepsilon_i(\lambda) C_i,$$

In Mathematical expression 2 above, $\mu_a$ may represent absorption coefficient (a total light absorption degree), $\varepsilon_i$ may represent absorption coefficient of each component (a skin component), and $C_i$ may represent concentration of each component (a skin component).

The skin component amount calculation unit 104 may calculate the total light absorption degree for light emitted from a light source on the basis of a volume fraction of hemoglobin component in the total volume of a component, a volume fraction of water and lipid components in the total volume of the component, a ratio of the water to lipid (water/(water + lipid)), and oxygen saturation of hemoglobin.

The skin component amount calculation unit 104 may calculate the total light absorption degree through Mathematical expression 3 below.

[Mathematical expression 3]

$$\mu_a(\lambda) = S_B\left((1 - SO_2)\mu_{a,HHb}(\lambda) + SO_2\mu_{a,HbO_2}(\lambda)\right) + S_{W+L}(R_{W+L}\mu_w\lambda + (1 - R_{W+L})\mu_L\lambda)$$

In Mathematical expression 3 above, $S_B$ may represent a volume fraction of a hemoglobin component in the total volume of a component, $S_{W+L}$ may represent a volume fraction of water and lipid components in the total volume of the component, $R_{W+L}$ may represent a ratio of water to lipid (water/(water + lipid)), and $SO_2$ may represent oxygen saturation, which may mean a ratio of oxygenated hemoglobin to total hemoglobin (unoxygenated hemoglobin + oxygenated hemoglobin).

The skin component amount calculation unit 104 may calculate a light signal (amount) detected by the light detector after energy is lost through absorption and scattering inside the skin by using the following Mathematical expression 4.

[Mathematical expression 4]

$$R[\mu_a(\lambda), \mu_s'(\lambda), \rho] = \frac{\mu_s'}{4\pi(\mu_s' + \mu_a)}\left[z_0\left(\mu_{eff} + \frac{1}{\tilde{r}_1}\right)\frac{exp(-\mu_{eff}\tilde{r}_1)}{\tilde{r}_1^2}\right.$$

$$\left. + (z_0 + 2z_b)\left(\mu_{eff} + \frac{1}{\tilde{r}_2}\right)\frac{exp(-\mu_{eff}\tilde{r}_2)}{\tilde{r}_2^2},\right]$$

$$\mu_{eff} = [3\,\mu_a(\mu_a + \mu_s')]^{1/2}$$

$$z_0 = (\mu_a + \mu_s')^{-1}$$

$$z_b = 2AD$$

$$D = 1/(3(\mu_a + \mu_s'))$$

$$\tilde{r}_1 = (z_0^2 + \rho^2)^{1/2}$$

$$\tilde{r_2} = ((z_0 + 2z_b)^2 + \rho^2)^{1/2}$$

In Mathematical expression 4 above, R may be a detected light signal, $\rho$ may be a distance between the light source and the light detector, and $\mu_{eff}$ may be effective diffusion coefficient, which may be a degree to which energy is actually reduced in a composite manner by absorption and scattering. D is diffusion coefficient, and A is a degree (an internal reflection factor) to which light is reflected inward at a boundary surface, and may be set to 1.

The skin component amount calculation unit 104 may calculate a skin component amount of a skin component through a fitting (e.g., least square curved fitting) process for a light signal (a light amount) calculated through Mathematical expression 4 for each of a plurality of wavelengths.

In an embodiment, the skin component amount calculation unit 104 may determine dummy data (or unnecessary (omitted) data) as part of component data obtained from the skin measurement unit 101. The skin component amount calculation unit 104 may calculate a skin component amount on the basis of component data excluding the determined dummy data. In other words, when a user runs or makes excessive motions, it may occur that the light source is not in close contact with the skin but is slightly away therefrom. In this case, light emitted from the light source leaks out and intensity thereof is reduced, so measurement based on this may be inaccurate and inconsistent. Therefore, the skin component amount calculation unit 104 may not use data for the time at which the light source is not in close contact with the skin.

In order for the skin component amount calculation unit 104 to determine the dummy data, the skin measurement unit 101 may determine a time point at which data is omitted. The skin measurement unit 101 may determine a user movement in a skin region of a user to which the skin measurement unit 101 is attached on the basis of data acquired from the acceleration sensor, the gyro sensor and/or the illuminance sensor included in the device 100, may determine a first time point at which the degree of the user movement is greater than or equal to the degree of a preset movement as the data omission time point, and may determine a second time point at which the degree of the user movement re-enters below the degree of the preset movement as a data use time point.

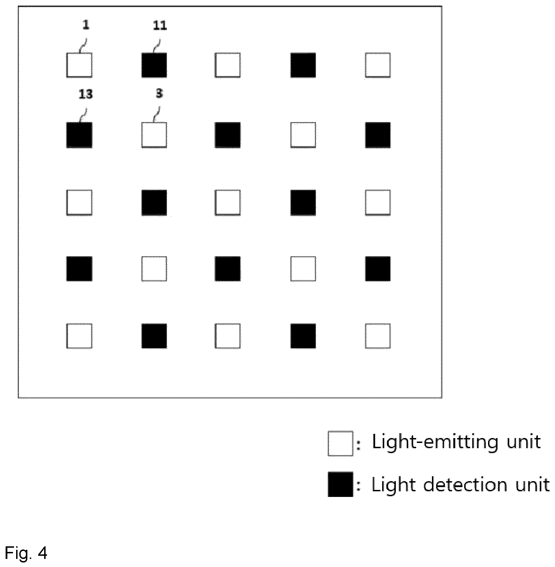

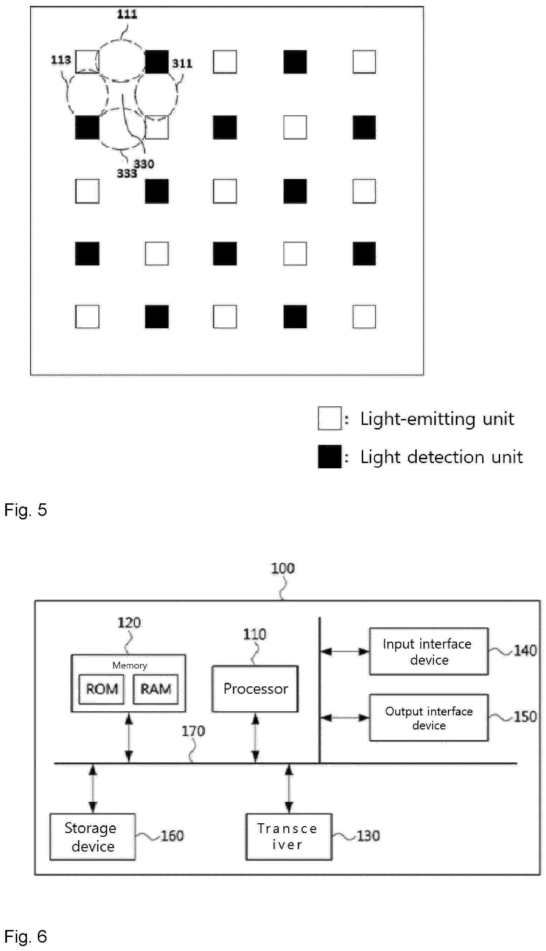

FIG. 3 is a drawing illustrating the light-emitting unit and the light detection unit in contact with the skin according to an embodiment. FIG. 4 is a diagram illustrating the arrangement of the light-emitting unit and the light detection unit of the skin measurement unit according to an embodiment. FIG. 5 is a diagram illustrating a region in which skin component data is detected according to the arrangement of the light-emitting unit and the light detection unit.

In an embodiment, referring to FIG. 3, the skin measurement unit 101 may calculate the amount of skin components by using a single light source and a single detector in the manner described through FIG. 2. The skin measurement unit 101 may calculate the amount of skin components by using an average optical characteristic value of a path between the light source and the light detector within the skin. A white light LED covering a visible light region may be used as a light detector in which a light source and a sensor are configured in an array of bandpass filter cells of different wavelengths, allowing a plurality of wavelengths to be detected at once. A first light source is LED white light, and the LED white light may include multiple wavelengths.

In an embodiment, referring to FIGS. 4 and 5, the skin component amount calculation unit 104 may generate a two-dimensional image map through an arrangement between multiple light sources and multiple light detectors. The skin measurement unit 101 may be configured in a checkerboard-like arrangement with each light source surrounded by light detectors from four directions. The skin component amount calculation unit 104 may generate spatial information for each skin component by filling gaps between measurement points through interpolation and generating a two-dimensional image map for each major skin component.

The skin component amount calculation unit 104 may measure a first component amount of a first skin region 111 between the first light source 1 and a first light detector 11 through the first light source 1 and the first light detector 11. The skin component amount calculation unit 104 may measure a second component amount of a second skin region 113 between the first light source 1 and a second light detector 13 through the first light source 1 and the second light detector 13. The skin component amount calculation unit 104 may measure a third component amount of a third skin region 311 between a second light source 3 and the first light detector 11 through the second light source 3 and the first light detector 11. The skin component amount calculation unit 104 may measure a fourth component amount of a fourth skin region 333 between the second light source 3 and the second light detector 13 through the second light source 3 and the second light detector 13.

The skin component amount calculation unit 104 may determine a fifth skin region 330 excluding the first skin region 111 to the fourth skin region 333. Alternatively, the skin component amount calculation unit 104 may determine an overlapping portion as the fifth skin region 330 when the first skin region 111 to the fourth skin region 333 overlap. The skin component amount calculation unit 104 may perform interpolation for the fifth skin region 330 on the basis of the first component amount to the fourth component amount, and determine a fifth component amount for the fifth skin region 330. When determining the fifth component amount, the skin component amount calculation unit 104 may determine a different weight for each of the first component amount to the fourth component amount. The skin component amount calculation unit 104 may calculate an average value of the first component amount to the fourth component amount, and may calculate a first difference value which is a difference between the calculated average value and the first component amount, a second difference value which is a difference between the average value and the second component amount, a third difference value which is a difference between the average value and the third component amount, and a fourth difference value which is a difference between the average value and the fourth component amount. The skin component amount calculation unit 104 may apply a weight higher than a preset weight to a component amount higher than the average value, and apply a weight lower than the preset weight to a component amount lower than the average value, and may apply a weight according to a ratio between the first difference value to the fourth difference value. For example, when the first component amount to the third component amount are higher than the average value and the fourth component amount is lower than the average value, the skin component amount calculation unit 104 may apply a higher weight for each of the first component amount to the third component amount than the preset weight, and may apply a higher weight as a magnitude difference between the first difference value to the third difference value increases and a lower weight as the magnitude difference decreases, and may apply a lower weight for the fourth component amount than the preset weight.

The skin component amount calculation unit 104 may generate a component amount distribution image (e.g., a melanin distribution image) for a specific component on the basis of the first component amount to the fifth component amount. The skin component amount calculation unit 104 may provide the generated component amount distribution image to a user.

FIG. 6 is a diagram illustrating the configuration of the hardware of the component amount measurement device 100 according to FIG. 1.

Referring to FIG. 6, the component amount measurement device 100 may include at least one processor 110 and a memory that stores instructions directing the at least one processor 110 to perform at least one operation.

The at least one operation may include at least some of the operations or functions of the component amount measurement device 100 described above and may be implemented in the form of instructions and performed by the processor 110.

Here, the at least one processor 110 may refer to a central processing unit (CPU), a graphics processing unit (GPU), or a dedicated processor on which methods according to the embodiments of the present disclosure are performed. Each of a memory 120 and a storage device 160 may be configured as at least one of a volatile storage medium and a non-volatile storage medium. For example, the memory 120 may be one of a read only memory (ROM) and a random access memory (RAM), and the storage device 160 may be a flash memory, a hard disk drive (HDD), a solid state drive (SSD), or various memory cards (e.g., a micro SD card), etc.

Additionally, the component amount measurement device 100 may include a transceiver 130 that performs communication via a wireless network. In addition, the component amount measurement device 100 may further include an input interface device 140, an output interface device 150, the storage device 160, etc. Each component included in the component amount measurement device 100 may be connected to each other by a bus 170 to communicate with each other. FIG. 6 illustrates the component amount measurement device 100 as an example, but is not limited thereto. For example, a plurality of user terminals may include the components of FIG. 6.

The methods according to the present disclosure may be implemented in the form of program instructions that can be executed through various computer means and may be recorded in a computer-readable medium. The computer-readable medium may include program instructions, data files, data structures, etc., either alone or in combination. The program instructions recorded in the computer-readable medium may be specially designed and configured for the present disclosure or may be known and available to those skilled in the art of computer software.

Examples of computer-readable media may include a hardware device specifically configured to store and execute program instructions, such as ROM, RAM, flash memory, and the like. Examples of the program instructions may include high-level language codes that can be executed by a computer by using an interpreter, etc. as well as machine language codes, such as those produced by a compiler. The hardware device described above may be configured to operate with at least one software module to perform the operations of the present disclosure, and vice versa.

In addition, the above-described method or device may be implemented by combining all or part of configuration or function thereof, or may be implemented by separating them.

Although the present disclosure has been described above with reference to preferred embodiments thereof, it will be understood by those skilled in the art that various modifications and changes may be made to the present disclosure without departing from the spirit and scope of the present disclosure as set forth in the claims below.

The component amount measurement device according to various embodiments disclosed in this document may be a device of various forms. The component amount measurement device may include, for example, a portable communication device (e.g., a smartphone), a computer device, a portable multimedia device, a portable medical device, a camera, a wearable device, or a consumer electronic device. The component amount measurement device according to the embodiment of this document is not limited to the devices described above.

It should be understood that the various embodiments of this document and the terms used herein are not intended to limit the technical features described in this document to specific embodiments, but rather encompass various modifications, equivalents, or substitutes of the embodiments. In connection with the description of the drawings, similar reference numerals may be used for similar or related components. The singular form of a noun corresponding to an item may include one or more of the items, unless the context clearly indicates otherwise. In this document, each of phrases such as "A or B", "at least one of A and B", "at least one of A or B", "A, B, or C", "at least one of A, B, and C", and "at least one of A, B, or C" may include any one of items listed together in the corresponding phrase among the phrases, or all possible combinations thereof. Terms such as "primary", "secondary", or "first" or "second" may be used merely to distinguish one component from another, and do not limit the components in any other respect (e.g., importance or order). When a component (e.g., a first component) is referred to as being "coupled" or "connected" to another component (e.g., a second component), with or without terms "functionally" or "communicatively," it means that the component may be connected to the another component directly (e.g., wired), wirelessly, or through a third component.

The term "module" as used in various embodiments of this document may include a unit implemented in hardware, software or firmware, and may be used interchangeably with terms such as logic, a logic block, component, or a circuit. A module may be a component that is configured integrally, a minimum unit of the component that performs one or more functions, or a part thereof. For example, according to an embodiment, a module may be implemented in the form of an application-specific integrated circuit (ASIC).

Various embodiments of the present document may be implemented as software (e.g., the program 240) including one or more instructions stored in a storage medium (e.g., internal memory 236 or external memory 238) readable by a machine (e.g., the component amount measurement device 100). For example, the processor (e.g., the processor 220) of the device (e.g., the component amount measurement device 100) may recall at least one instruction from among one or more instructions stored from the storage medium and execute it. This enables the device to be operated to perform at least one function in response to the at least one instruction recalled above. The one or more of the instructions may include a code generated by a compiler or a code which can be executed by an interpreter. The device-readable storage medium may be provided in the form of a non-transitory storage medium. Here, the term "non-transitory" simply means that the storage medium is a tangible device and does not include signals (e.g. electromagnetic waves), and does not distinguish between cases in which data is stored semi-permanently or temporarily in the storage medium.

According to an embodiment, the method according to various embodiments disclosed in the present document may be provided as part of a computer program product. The computer program product as a commodity may be traded between a seller and a buyer. The computer program product may be distributed in the form of a machine-readable storage medium (e.g., a compact disc read only memory (CD-ROM)), or may be distributed online (e.g., by download or upload) via an application store (e.g., Play Store ™) or directly between two user devices (e.g., smart phones). In the case of online distribution, at least a portion of the computer program product may be temporarily stored or generated in a machine-readable storage medium, such as the memory of a server of a manufacturer, a server of an application store, or an intermediary server.

According to various embodiments, each component (e.g., a module or a program) of the above-described components may include a single entity or a plurality of entities, and some of the plurality of entities may be arranged separately from other components. According to various embodiments, one or more components or operations of the aforementioned components may be omitted, or one or more other components or operations may be added. Alternatively or additionally, a plurality of components (e.g., modules or programs) may be integrated into a single component. In such a case, the integrated component may perform one or more functions of each of the plurality of components identically or similarly to those performed by the corresponding component among the plurality of components prior to the integration. According to various embodiments, operations performed by a module, program or other component may be performed sequentially, in parallel, iteratively, or heuristically, or one or more of the operations may be performed in a different order, omitted, or one or more other operations may be added.

## Claims

1.    A component amount measurement device for measuring skin component amount information about a user, the device comprising:

a skin measurement unit for measuring the skin of the user; and
a skin component amount calculation unit for calculating a component amount for a target component in the skin of the user,
wherein the skin measurement unit comprises:

a light-emitting unit that comprises at least one light source; and a light detection unit that comprises at least one light detector,
wherein the light-emitting unit comprises a first light source for irradiating a surface of the skin with a first light having a plurality of wavelengths; and the light detection unit comprises a first light detector for detecting the first light emitted from the first light source,
wherein the skin component amount calculation unit measures the component amount for the target component on the basis of a light signal of the first light absorbed and scattered within the skin.

2. The device of claim 1, wherein light emitted from the at least one light source is LED white light,

the first light detector comprises a plurality of bandpass filters arranged in a grid arrangement, and obtains a light signal for each of the plurality of wavelengths comprised in the first light through the plurality of bandpass filters, and
the skin component amount calculation unit calculates the component amount for the target component on the basis of the light signal for each of the plurality of wavelengths.

3. The device of claim 2, wherein the skin component amount calculation unit calculates the component amount for the target component on the basis of at least concentration of each component in the skin, extinction coefficient of each component in the skin, and a distance between the first light source and the first light detector.

4. The device of claim 2, wherein the at least one light source comprises the first light source and a second light source arranged to be adjacent to the first light source, and the at least one light detector comprises the first light detector and a second light detector arranged to be adjacent to the first light detector,

the skin component amount calculation unit calculates a first component amount for the target component within a first skin region between the first light source and the first light detector through the first light source and the first light detector,
calculates a second component amount for the target component within a second skin region between the first light source and the second light detector through the first light source and the second light detector,
calculates a third component amount for the target component within a third skin region between the second light source and the first light detector through the second light source and the first light detector, and
calculates a fourth component amount for the target component within a fourth skin region between the second light source and the second light detector through the second light source and the second light detector.

5. The device of claim 4, wherein the skin component amount calculation unit determines a fifth skin region excluding the first skin region to the fourth skin region,

calculates a fifth component amount for the target component within the fifth skin region on the basis of the first component amount to the fourth component amount, and
generates a two-dimensional image map for the target component to generate spatial information for the target component.

Fig. 1

**100**

Skin measurement unit — 101

Light-emitting unit — 102

Light detection unit — 103

Skin component amount calculation unit — 104

Fig. 2

☐ : Light-emitting unit

■ : Light detection unit

Fig. 3

□: Light-emitting unit

■: Light detection unit

Fig. 4

□ : Light-emitting unit

■ : Light detection unit

Fig. 5

Fig. 6

<table>
<tr><td colspan="2">**INTERNATIONAL SEARCH REPORT**</td><td>International application No.<br>**PCT/KR2023/009860**</td></tr>
</table>

**A.  CLASSIFICATION OF SUBJECT MATTER**

**A61B 5/00**(2006.01)i

According to International Patent Classification (IPC) or to both national classification and IPC

**B.  FIELDS SEARCHED**

Minimum documentation searched (classification system followed by classification symbols)

A61B 5/00(2006.01); A61B 5/08(2006.01); A61B 5/117(2006.01); G06K 9/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 피부 성분량 측정(skin components measurement), 광원(light source), 광 검출(light detection), 다파장(multiple wavelengths), 밴드패스 필터(bandpass filter)

**C.  DOCUMENTS CONSIDERED TO BE RELEVANT**

| Category* | Citation of document, with indication, where appropriate, of the relevant passages | Relevant to claim No. |
|---|---|---|
| X | KR 10-2019-0081634 A (SAMSUNG ELECTRONICS CO., LTD.) 09 July 2019 (2019-07-09)<br>See paragraphs [0040], [0055]-[0056], [0064], [0094], [0115], [0118], [0121] and [0148]; and claims 1 and 5-6. | 1 |
| Y | | 2-3 |
| A | | 4-5 |
| Y | KR 10-2021-0075648 A (SAMSUNG ELECTRONICS CO., LTD.) 23 June 2021 (2021-06-23)<br>See abstract; and paragraphs [0043]-[0044] and [0059]. | 2-3 |
| Y | KR 10-2021-0050967 A (SAMSUNG ELECTRONICS CO., LTD.) 10 May 2021 (2021-05-10)<br>See paragraphs [0040]-[0041], [0055] and [0146]. | 3 |
| A | KR 10-2022-0010890 A (SAMSUNG ELECTRONICS CO., LTD.) 27 January 2022 (2022-01-27)<br>See entire document. | 1-5 |

☑ Further documents are listed in the continuation of Box C.     ☑ See patent family annex.

| | | | |
|---|---|---|---|
| \* | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 October 2023** | **12 October 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/009860**

**C.    DOCUMENTS CONSIDERED TO BE RELEVANT**

| Category* | Citation of document, with indication, where appropriate, of the relevant passages | Relevant to claim No. |
|---|---|---|
| A | US 2016-0120468 A1 (KONINKLIJKE PHILIPS N.V.) 05 May 2016 (2016-05-05)<br>See entire document. | 1-5 |
| PX | KR 10-2457772 B1 (LULULAB INC.) 24 October 2022 (2022-10-24)<br>See claims 1 and 3.<br>(※This document is the published patent of a priority application of the present international application.) | 1-5 |

Form PCT/ISA/210 (second sheet) (July 2022)

| | INTERNATIONAL SEARCH REPORT<br>Information on patent family members | | | | International application No.<br>**PCT/KR2023/009860** | |

| Patent document<br>cited in search report | | | Publication date<br>(day/month/year) | Patent family member(s) | | | Publication date<br>(day/month/year) |
|---|---|---|---|---|---|---|---|
| KR | 10-2019-0081634 | A | 09 July 2019 | CN | 109984727 | A | 09 July 2019 |
| | | | | CN | 109984727 | B | 02 June 2023 |
| | | | | EP | 3505044 | A1 | 03 July 2019 |
| | | | | EP | 3505044 | B1 | 09 September 2020 |
| | | | | JP | 2019-120684 | A | 22 July 2019 |
| | | | | JP | 7152287 | B2 | 12 October 2022 |
| | | | | KR | 10-2531994 | B1 | 15 May 2023 |
| | | | | US | 11013412 | B2 | 25 May 2021 |
| | | | | US | 11607133 | B2 | 21 March 2023 |
| | | | | US | 2019-0200866 | A1 | 04 July 2019 |
| | | | | US | 2021-0235990 | A1 | 05 August 2021 |
| KR | 10-2021-0075648 | A | 23 June 2021 | CN | 112971777 | A | 18 June 2021 |
| | | | | EP | 3835763 | A1 | 16 June 2021 |
| | | | | US | 11530948 | B2 | 20 December 2022 |
| | | | | US | 2021-0181019 | A1 | 17 June 2021 |
| | | | | US | 2023-0055190 | A1 | 23 February 2023 |
| KR | 10-2021-0050967 | A | 10 May 2021 | CN | 112741624 | A | 04 May 2021 |
| | | | | EP | 3815608 | A1 | 05 May 2021 |
| | | | | US | 2021-0121102 | A1 | 29 April 2021 |
| KR | 10-2022-0010890 | A | 27 January 2022 | CN | 113951814 | A | 21 January 2022 |
| | | | | EP | 3942998 | A1 | 26 January 2022 |
| | | | | US | 11717168 | B2 | 08 August 2023 |
| | | | | US | 2022-0015641 | A1 | 20 January 2022 |
| US | 2016-0120468 | A1 | 05 May 2016 | CN | 107106051 | A | 29 August 2017 |
| | | | | CN | 107106051 | B | 23 June 2020 |
| | | | | EP | 3212060 | A1 | 06 September 2017 |
| | | | | US | 10231667 | B2 | 19 March 2019 |
| | | | | WO | 2016-066841 | A1 | 06 May 2016 |
| KR | 10-2457772 | B1 | 24 October 2022 | | None | | |

Form PCT/ISA/210 (patent family annex) (July 2022)